# EUROPEAN PATENT APPLICATION

(11) **EP 4 790 052 A1**
(43) Date of publication of application: **12.08.2026**
(21) Application number: 24874734.7
(22) Date of filing: 04.10.2024
(51) Int. Cl.: C07D 311/94, C09K 9/02, G02B 5/23, G02C 7/10

(54) **PHOTOCHROMIC COMPOUND, PHOTOCHROMIC COMPOSITION, PHOTOCHROMIC ARTICLE, AND EYEGLASSES**

(30) Priority: 06.10.2023 JP 2023174462
(71) Applicant: HOYA LENS THAILAND LTD., Pathumthani 12130 (TH)
(72) Inventor: KAWAKAMI, Hironori, Tokyo 160-8347 (JP); KOBAYASHI, Kei, Tokyo 160-8347 (JP)
(74) Representative: Hoffmann Eitle
(86) International application number: PCT/JP2024/035582
(87) International publication number: WO 2025/075134

(57) **Abstract**

A photochromic compound represented by General Formula 1 is provided. In General Formula 1, R¹ and R² are bonded together to form a ring structure, the ring structure being a ring structure spiro-condensed with an indeno-condensed naphthopyran, or R¹ and R² each independently represent a substituted or unsubstituted, linear or branched alkyl group having 1 or more carbon atoms; a substituted or unsubstituted cyclic alkyl group having 3 or more carbon atoms; or a substituted or unsubstituted group represented by - (R¹⁰⁰)ₙR¹⁰¹, where R¹⁰⁰ represents an alkyleneoxy group, R¹⁰¹ represents an alkyl group, n represents an integer of 1 or more, and the total number of carbon atoms in n alkyleneoxy groups represented by R¹⁰⁰ and an alkyl group represented by R¹⁰¹ is 2 or more; R³ to R⁶ each independently represent a hydrogen atom or a non-conjugated substituent; R⁷ to R¹⁰ each independently represent a hydrogen atom or a substituent; R¹¹ to R²⁰ each independently represent a hydrogen atom or a substituent, provided that at least one of R¹¹ to R²⁰ represents an amino group represented by General Formula 2 below. In General Formula 2, n represents 0 or 1; when n is 0, Ar represents a substituted or unsubstituted aryl group having 5 or more ring constituent atoms, or a substituted or unsubstituted heteroaryl group having 5 or more ring constituent atoms, and when n is 1, Ar represents a substituted or unsubstituted arylene group having 5 or more ring constituent atoms, or a substituted or unsubstituted heteroarylene group having 5 or more ring constituent atoms; when n is 0, R represents a monovalent substituent, and when n is 1, R represents a divalent substituent; L is a single bond or a divalent linking group selected from the group consisting of a substituted or unsubstituted, linear, branched, or cyclic alkylene group having 1 to 10 carbon atoms, a substituted or unsubstituted arylene group having 1 to 20 carbon atoms, a substituted or unsubstituted alkylarylene group having 1 to 20 carbon atoms, an ether group, a sulfide group, a sulfone group, a sulfoxide group, -NR²⁰⁰- (where R²⁰⁰ represents a hydrogen atom or a substituent), and -SiRaRb - (where Ra and Rb each independently represent a substituted or unsubstituted alkyl group); and * represents a bonding position with a benzene ring.

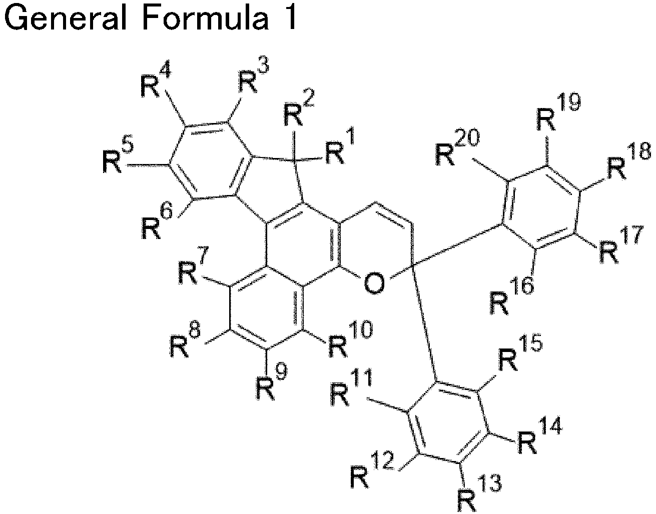

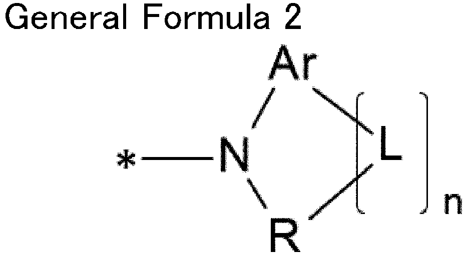

## Description

### Technical Field

The present invention relates to a photochromic compound, a photochromic composition, a photochromic article, and spectacles.

### Background Art

Photochromic compounds are compounds that exhibit properties (photochromic properties), coloring under irradiation with light in the wavelength range for which the photochromic compounds become photosensitive and fading when not irradiated. For example, PTL 1 to 3 discloses a naphthopyran compound having photochromic properties.

### Citation List

### Patent Literature

PTL 1: WO 2001/60811 A1
PTL 2: WO 1996/014596 A1
PTL 3: U.S. Patent Specification No. 6296785

### Summary of Invention

### Technical Problem

As a method of imparting photochromic properties to various articles, a method of incorporating a photochromic compound into a substrate and a method of forming a layer containing a photochromic compound may be exemplified. For example, an article (photochromic article) to which photochromic properties are imparted as such is suitable as an optical article, such as a spectacle lens.

As an example, a photochromic compound is irradiated with light, such as sunlight, and undergoes structural conversion into a colored form through an excited state. The structure after structural conversion through irradiation with light may be called a "colored form". In contrast, the structure before irradiation with light may be called a "colorless form". Here, regarding the colorless form, the term "colorless" is not limited to being completely colorless, and includes a case in which the color is lighter than that of the colored form. A photochromic compound that shows small absorption of visible light having a short wavelength around 400 nm in the state of the colorless form is preferable for providing a photochromic compound that is less colored (for example, less yellowish) in a usage environment, such as indoors.

In recent years, research has been conducted on achieving desired color tones during the coloring of photochromic articles by combining a plurality of photochromic compounds having different absorption characteristics. As such a photochromic compound, a photochromic compound having a large absorption peak on the longer wavelength side (for example, at or above the wavelength of 580 nm) is useful. However, in the photochromic compound of the structure in which a colored form has a large absorption peak on the longer wavelength side, not only the colored form but also the uncolored form tend to absorb light on the longer wavelength side, and absorption of visible light having a short wavelength around 400 nm tends to be large in the state of the colorless form.

An object of one aspect of the present invention is to provide a photochromic compound having a large absorption peak on the longer wavelength side and showing small absorption of short-wavelength visible light in the state of the colorless form.

### Solution to Problem

The present inventors have conducted extensive studies, and as a result, have newly found that a photochromic compound represented by General Formula 1 having a structure shown below has a peak wavelength on the longer wavelength side and shows small absorption of a short-wavelength visible light in the state of the colorless form.

In other words, one aspect of the present invention relates to a photochromic compound represented by General Formula 1 below.

The structure of General Formula 1 is a structure of the colorless form.

In addition, one aspect of the present invention relates to a photochromic article including at least one photochromic compound represented by General Formula 1 below.

In addition, one aspect of the present invention relates to a photochromic composition including at least one photochromic compound represented by General Formula 1 below.

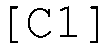

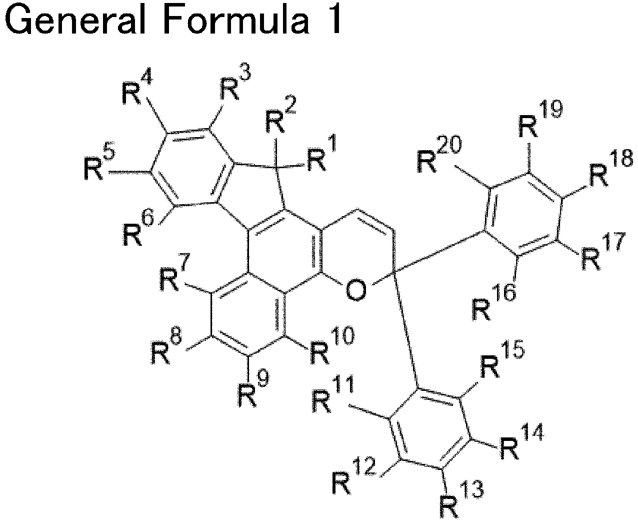

In General Formula 1,
R¹ and R² are bonded together to form a ring structure, the ring structure being a ring structure spiro-condensed with an indeno-condensed naphthopyran, or
R¹ and R² each independently represent a substituted or unsubstituted, linear or branched alkyl group having 1 or more carbon atoms; a substituted or unsubstituted cyclic alkyl group having 3 or more carbon atoms; or a substituted or unsubstituted group represented by -(R¹⁰⁰)ₙR¹⁰¹, where R¹⁰⁰ represents an alkyleneoxy group, R¹⁰¹ represents an alkyl group, n represents an integer of 1 or more, and the total number of carbon atoms in n alkyleneoxy groups represented by R¹⁰⁰ and an alkyl group represented by R¹⁰¹ is 2 or more;
R³ and R⁶ each independently represent a hydrogen atom or a non-conjugated substituent;
R⁷ to R¹⁰ each independently represent a hydrogen atom or a substituent;
R¹¹ to R²⁰ each independently represent a hydrogen atom or a substituent, provided that at least one of R¹¹ to R²⁰ represents an amino group represented by General Formula 2 below.

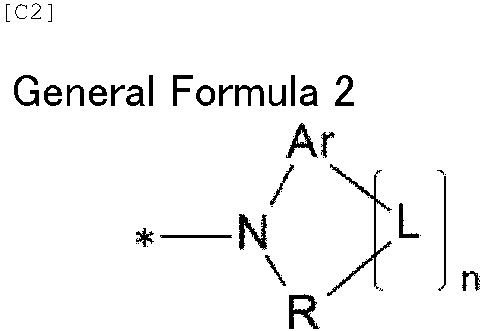

In General Formula 2,
n represents 0 or 1;
when n is 0, Ar represents a substituted or unsubstituted aryl group having 5 or more ring constituent atoms, or a substituted or unsubstituted heteroaryl group having 5 or more ring constituent atoms, and when n is 1, Ar represents a substituted or unsubstituted arylene group having 5 or more ring constituent atoms, or a substituted or unsubstituted heteroarylene group having 5 or more ring constituent atoms;
when n is 0, R represents a monovalent substituent, and when n is 1, R represents a divalent substituent;
L represents a single bond or a divalent linking group selected from the group consisting of a substituted or unsubstituted, linear, branched, or cyclic alkylene group having 1 to 10 carbon atoms, a substituted or unsubstituted arylene group having 1 to 20 carbon atoms, a substituted or unsubstituted alkylarylene group having 1 to 20 carbon atoms, an ether group, a sulfide group, a sulfone group, a sulfoxide group, -NR¹⁰⁰- (where R¹⁰⁰ represents a hydrogen atom or a substituent), and -SiRaRb - (where Ra and Rb each independently represent a substituted or unsubstituted alkyl group); and
* represents a bonding position with a benzene ring. Advantageous Effects of Invention

According to one aspect of the present invention, it is possible to provide a photochromic compound having a large absorption peak on the longer wavelength side and showing small absorption of a short wavelength visible light in the state of the colorless form, and a photochromic article containing the photochromic compound.

### Description of Embodiments

In the present invention and this specification, the term "photochromic article" shall refer to an article containing a photochromic compound. A photochromic article according to one aspect of the present invention at least contains at least one photochromic compound represented by General Formula 1 as a photochromic compound. The photochromic compound may be incorporated into a substrate of a photochromic article and/or may be incorporated into a photochromic layer in a photochromic article having a substrate and a photochromic layer. The term "photochromic layer" refers to a layer containing a photochromic compound.

In the present invention and this specification, the term "photochromic composition" shall refer to a composition containing a photochromic compound. A photochromic composition according to one aspect of the present invention at least contains at least one photochromic compound represented by General Formula 1 as a photochromic compound, and may be used to produce a photochromic article according to one aspect of the present invention.

In the present invention and the present specification, substituents in various general formulas to be described in detail below and substituents when each group to be described below has a substituent may each independently be the following:
a substituent R^{m} selected from the group consisting of a hydroxy group; a C1-18 linear or branched alkyl group, such as a methyl group, an ethyl group, a propyl group, a butyl group, a pentyl group, and a hexyl group; a monocyclic cycloaliphatic C5-18 alkylene group, such as a cyclopentylene group or a cyclohexylene group, or a polycyclic cycloaliphatic C5-18 alkylene group, such as a bicyclic ring; a linear or branched alkoxy group having 1 to 24 constituent atoms, such as a methoxy group, an ethoxy group, and a butoxy group; a nonaromatic cyclic substituent having 1 to 24 constituent atoms;
a C1-18 linear or branched perfluoroalkyl group, such as a trifluoromethyl group; a linear or branched perfluoroalkoxy group, such as a trifluoromethoxy group; a linear or branched alkyl sulfide group having 1 to 24 constituent atoms, such as a methyl sulfide group, an ethyl sulfide group, and a butyl sulfide group; an aryl group, such as a phenyl group, a naphthyl group, an anthracenyl group, a fluoranthenyl group, a phenanthryl group, a pyranyl group, a perylenyl group, a styryl group, and a fluorenyl group; an aryloxy group, such as a phenoxy group; an aryl sulfide group, such as a phenyl sulfide group; a heteroaryl group, such as a pyridyl group, a furanyl group, a thienyl group, a pyrrolyl group, a benzofuranyl group, a benzothiophenyl group, an indolyl group, a dibenzofuranyl group, a dibenzothiophenyl group, a carbazolyl group, a diazolyl group, a triazolyl group, a quinolinyl group, a phenothiazinyl group, a phenoxazinyl group, a phenazinyl group, a thianthryl group, and an acridinyl group; an amino group (-NH₂); a monoalkylamino group, such as a monomethylamino group; a dialkylamino group, such as a dimethylamino group; a monoarylamino group, such as a monophenylamino group; a diarylamino group, such as a diphenylamino group; a cyclic amino group, such as a piperidino group, a morpholino group, a thiomorpholino group, a tetrahydroquinolino group, and a tetrahydroisoquinolino group; an ethynyl group; a mercapto group; a silyl group; a sulfonic acid group; an alkylsulfonyl group; a formyl group; a carboxy group; a cyano group; and a halogen atom, such as a fluorine atom, a chlorine atom, a bromine atom, and an iodine atom; or
a substituent in which R^{m} is additionally substituted with at least one same or different R^{m}.

As an example of the substituent in which the above R^{m} is additionally substituted with at least one same or different R^{m}, a structure in which the terminal carbon atom of an alkoxy group is additionally substituted with another alkoxy group, and the terminal carbon atom of the alkoxy group is additionally substituted with another alkoxy group may be exemplified. In addition, as another example of the substituent in which the above R^{m} is additionally substituted with at least one same or different R^{m}, a structure in which two or more positions among five substitutable positions of a phenyl group are substituted with the same or different R^{m} may be exemplified. However, the present invention is not limited to such examples.

In the present invention and the present specification, the terms "the number of carbon atoms" and "the number of constituent atoms" shall refer to the numbers including the number of carbon atoms or the number of atoms in a substituent, when referring to a group having a substituent, unless otherwise specified. In addition, in the present invention and the present specification, the term "substituted or unsubstituted" has the same meaning as "unsubstituted or having at least one substituent".

In addition, in the present invention and this specification, substituents in various general formulas of which details will be described below, and substituents when respective groups to be described below have substituents may each independently be a solubilizing group. In the present invention and this specification, the term "solubilizing group" refers to a substituent that can contribute to increasing the compatibility with any liquid or a specific liquid. The solubilizing group is suitably a substituent that can contribute to promoting the thermal motion of molecules of a compound by possessing this substituent, and examples of solubilizing groups may include an alkyl group including a linear, branched, or cyclic structure having 4 to 50 carbon atoms; a linear, branched, or cyclic alkoxy group having 4 to 50 constituent atoms; a linear, branched, or cyclic silyl group having 4 to 50 constituent atoms, a group in which part of the above group is substituted with a silicon atom, a sulfur atom, a nitrogen atom, a phosphorus atom, or the like, and a group obtained by combining two or more of the above groups. A compound having a solubilizing group as a substituent can inhibit the distance between solute molecules from decreasing, thereby preventing the solute from solidifying, and can lower the melting point and/or glass transition temperature of the solute to form a molecule aggregation state close to that of a liquid. Therefore, the solubilizing group can liquefy a solute or increase the solubility of a compound having this substituent in a liquid. In one embodiment, the solubilizing group is preferably an n-butyl group, n-pentyl group, n-hexyl group, and n-octyl group, which are linear alkyl groups, a tert-butyl group, which is a branched alkyl group, or a cyclopentyl group and a cyclohexyl group, which are cyclic alkyl groups.

The substituent is preferably a substituent selected from the group consisting of a methoxy group, an ethoxy group, a phenoxy group, a methylsulfide group, an ethylsulfide group, a phenylsulfide group, a trifluoromethyl group, a phenyl group, a naphthyl group, a dibenzofuranyl group, a dibenzothiophenyl group, a carbazolyl group, a phenothiazinyl group, a phenoxazinyl group, a phenazinyl group, an acridinyl group, a dimethylamino group, a diphenylamino group, a piperidino group, a morpholino group, a thiomorpholino group, a cyano group, and a solubilizing group, and more preferably a substituent selected from the group consisting of a methoxy group, a phenoxy group, a methylsulfide group, a phenylsulfide group, a trifluoromethyl group, a phenyl group, a dimethylamino group, a diphenylamino group, a piperidino group, a morpholino group, a thiomorpholino group, a cyano group, and a solubilizing group.

In the present invention and the present specification, the term "electron-withdrawing group" refers to a substituent that tends to withdraw electrons from the side of atoms bonded thereto more easily, as compared to a hydrogen atom. Electron-withdrawing groups can withdraw electrons as a result of substituent effects, such as an inductive effect and a mesomeric effect (or a resonance effect). Specific examples of electron-withdrawing groups may include a halogen atom (a fluorine atom: -F, a chlorine atom: -Cl, a bromine atom: -Br, and an iodine atom: -I), a trifluoromethyl group: -CF₃, a nitro group: -NO₂, a cyano group: -CN, a formyl group: -CHO, an acyl group: -COR (where R is a substituent), an alkoxycarbonyl group: -COOR, a carboxy group: -COOH, a substituted sulfonyl group: -SO₂R (where R is a substituent), a sulfo group: -SO₃H, and the like. Examples of preferred electron-withdrawing groups may include a fluorine atom, which is an electron-withdrawing group having high electronegativity, and an electron-withdrawing group having a positive value for the substituent constant σₚ at the para-position based on Hammett's rule.

In the present invention and the present specification, the term "electron-donating group" refers to a substituent that tends to donate electrons to a bonding atom side more easily, as compared to a hydrogen atom. An electron-donating group may be a substituent that tends to donate electrons due to the sum of the inductive effect, the mesomeric effect (or resonance effect), and the like. Specific examples of electron-donating groups may include a hydroxy group: -OH, a thiol group: -SH, an alkoxy group: -OR (where R is an alkyl group), an alkylsulfide group: -SR (where R is an alkyl group), an arylsulfide group, an acetyl group: -OCOCH₃, an amino group: -NH₂, an alkylamino group: -NHR (where R is an alkyl group), an acetoamido group: -NHCOCH₃, a dialkylamino group: -N(R)₂ (where two Rs are the same or different alkyl groups), a methyl group, and the like. Examples of suitable electron-donating groups may include an electron-donating group having a negative value for the substituent constant σₚ at the para-position based on Hammett's rule.

Specific examples of the substituent constant σₚ at the para-position based on Hammett's rule (source: edited by Hiizu IWAMURA, Ryoji NOYORI, Takeshi NAKAI, and Isao KITAGAWA, Organic Chemistry for Graduate School (volume 1) (1988)) are shown below.
-N(CH₃)₂: -0.83
-OCH₃: -0.27
-t-C₄H₉: -0.20
-CH₃: -0.17
-C₂H₅: -0.15
-C₆H₅: -0.01
(-H: 0)
-F: +0.06
-Cl: +0.27
-Br: +0.23
-CO₂C₂H₅: +0.45
-CF₃: +0.54
-CN: +0.66
-SO₂CH₃: +0.72
-NO₂: +0.78

### [Photochromic Compound Represented by General Formula 1]

Hereinafter, the photochromic compound represented by General Formula 1 will be described in more detail.

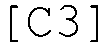

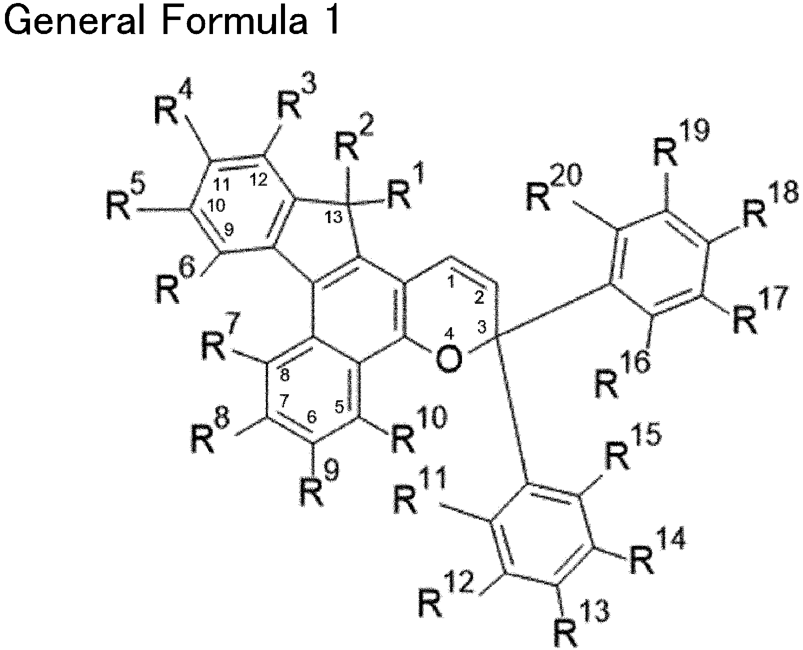

In General Formula 1,
R¹ and R² are bonded together to form a ring structure, the ring structure being a ring structure spiro-condensed with an indeno-condensed naphthopyran, or
R¹ and R² each independently represent a substituted or unsubstituted, linear or branched alkyl group having 1 or more carbon atoms; a substituted or unsubstituted cyclic alkyl group having 3 or more carbon atoms; or a substituted or unsubstituted group represented by -(R¹⁰⁰)ₙR¹⁰¹, where R¹⁰⁰ represents an alkyleneoxy group, R¹⁰¹ represents an alkyl group, n represents an integer of 1 or more, and the total number of carbon atoms in n alkyleneoxy groups represented by R¹⁰⁰ and an alkyl group represented by R¹⁰¹ is 1 or more.

In one embodiment, in General Formula 1, R¹ and R² each independently represent a substituted or unsubstituted, linear or branched alkyl group having 1 or more carbon atoms; a substituted or unsubstituted cyclic alkyl group having 3 or more carbon atoms; or a substituted or unsubstituted group represented by -(R¹⁰⁰)ₙR¹⁰¹, where R¹⁰⁰ represents an alkyleneoxy group, R¹⁰¹ represents an alkyl group, n represents an integer of 1 or more, and the total number of carbon atoms in n alkyleneoxy groups represented by R¹⁰⁰ and an alkyl group represented by R¹⁰¹ is 2 or more.

In General Formula 1, R¹ and R² each independently represents a substituted or unsubstituted, linear or branched alkyl group having a carbon number 1 or more (for example, 1 or more and 20 or less, preferably 1 or more and 10 or less); a substituted or unsubstituted a cyclic alkyl group having 3 or more (for example, 3 or more and 20 or less) carbon atoms; or a substituted or unsubstituted group represented by -(R¹⁰⁰)ₙR¹⁰¹, where R¹⁰⁰ represents an alkyleneoxy group; R¹⁰¹ represents an alkyl group; n represents an integer of 1 or more; and the total carbon number of the n alkyleneoxy groups represented by R¹⁰⁰ and the alkyl groups represented by R¹⁰¹ is 2 or more (for example, 2 or more and 50 or less). If the above linear or branched alkyl group having 1 or more carbon atoms has a substituent, the carbon number of the alkyl group refers to the carbon number of a portion excluding the substituent. If the cyclic alkyl group having 3 or more carbon atoms has a substituent, the carbon number of the alkyl group refers to the carbon number of a portion excluding the substituent. If the group represented by "-(R¹⁰⁰)ₙR¹⁰¹" has a substituent; the total carbon number of the n alkyleneoxy groups represented by R¹⁰⁰ and the alkyl groups represented by R¹⁰¹ refers to the carbon number of a portion excluding the substituent.

It is preferred that R¹ and R² each independently represent a methyl group, an ethyl group, a propyl group, an isopropyl group, a n-butyl group, a s-butyl group, or a t-butyl group. It is more preferred that R³⁰ and R³¹ both represent an ethyl group. In one embodiment, R¹ to R² may each independently represent a substituted or unsubstituted methyl group, a substituted or unsubstituted ethyl group, or a substituted or unsubstituted propyl group. In one embodiment, R¹ and R² may each independently be an unsubstituted methyl group, an unsubstituted ethyl group, or an unsubstituted propyl group. Also, in one embodiment, R¹ and R² may both be an unsubstituted methyl group, may both be an unsubstituted ethyl group, or may both an unsubstituted propyl group.

R¹ and R² may be the same or different groups.

In one embodiment, R¹ and R² are bonded together to form a ring structure, where this ring structure is a ring structure spiro-condensed with an indeno-condensed naphthopyran. Such a ring structure may be unsubstituted or may have a substituent. When the ring structure has a ring structure having a substituent, the number of carbon atoms described above shall refer to the number of carbon atoms including the number of carbon atoms in the substituent. The number of carbon atoms in the ring structure described above (including the carbon atom at the 13-position of the indeno-condensed naphthopyran) may be 3 or more, and may also be 4 or more, 5 or more, 6 or more, or 7 or more. In addition, the number of carbon atoms in the ring structure described above (including the carbon atom at the 13-position of the indeno-condensed naphthopyran) may be 20 or less, and may also be 19 or less, 18 or less, 17 or less, 16 or less, or 15 or less.

The ring structure described above may be a substituted or unsubstituted alicyclic structure. Such an alicyclic structure may be a monocyclic structure, may be a condensed polycyclic structure, such as a bicyclic or tricyclic structure, or may be a bridged ring structure, such as a bicyclic structure.

In General Formula 1, specific examples of the following partial structure: 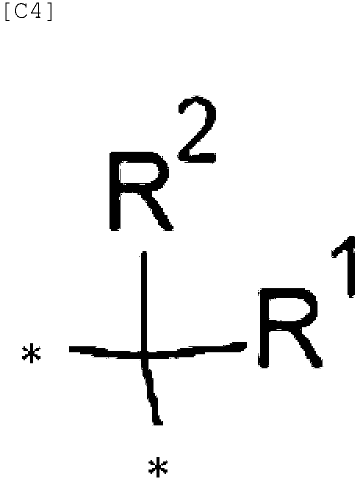 may include the following partial structures and partial structures included in exemplary compounds listed below. In the present invention and the present specification, the symbol "*" for a partial structure of a compound indicates a bonding position with an atom to which such a partial structure is bonded.

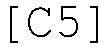

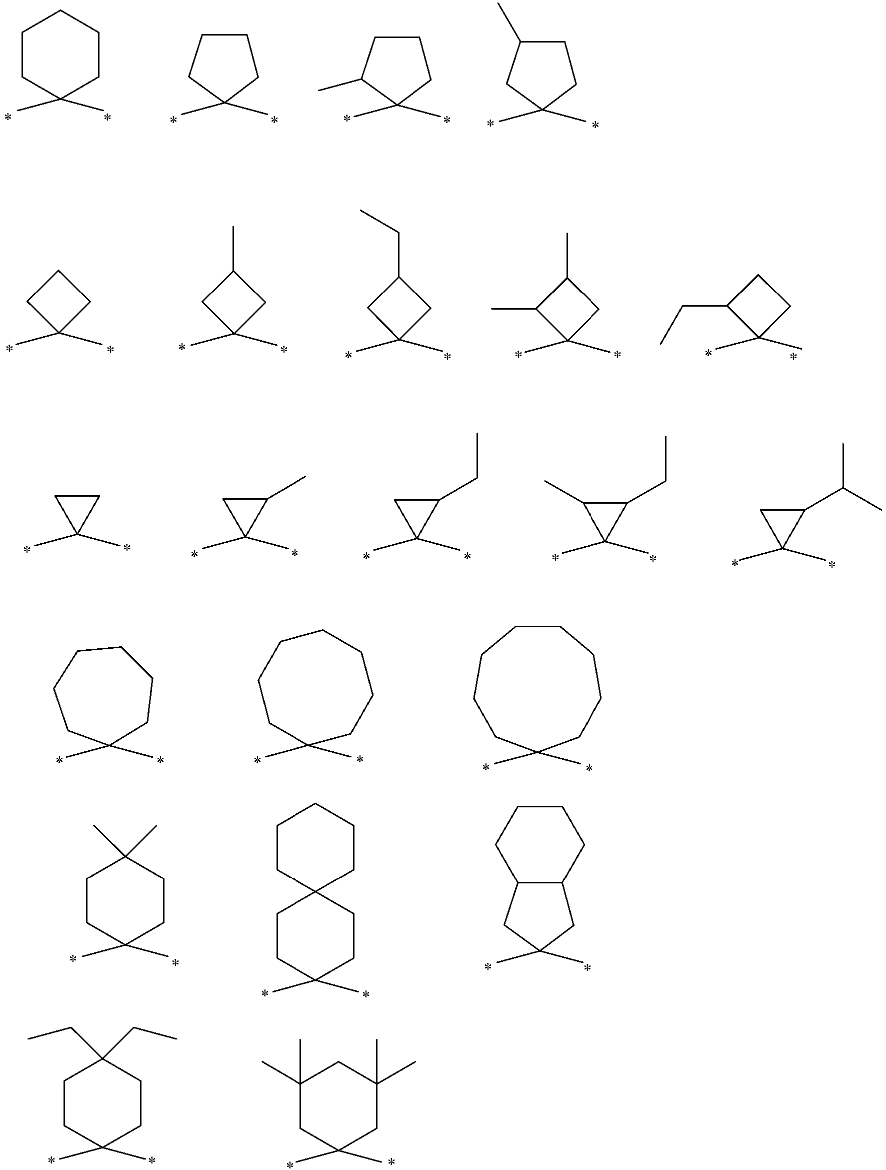

R³ to R⁶ each independently represent a hydrogen atom or a non-conjugated substituent. In contrast, if R³ to R⁶ (in particular, R⁴) is substituted with a substituent other than a conjugated substituent, the absorption of short-wavelength visible light by a photochromic compound tends to increase.

In the present invention and the present specification, the term "non-conjugated substituent" shall refer to a substituent which, when substituted to an aromatic moiety, does not form a conjugated system with an aromatic ring. The non-conjugated substituent preferably satisfies at least one of the following conditions (i) to (iii), more preferably at least two thereof, and still more preferably all three:
(i) atoms constituting this substituent are only sp3 hybridized atoms.
(ii) atoms constituting this substituent do not possess a lone pair.
(iii) the substituent does not possess empty p orbitals.

Specific examples of non-conjugated substituents may include a substituted or unsubstituted alkyl group, such as a substituted or unsubstituted methyl, ethyl, or propyl group, a substituted or unsubstituted silyl group, a halogen atom, such as a fluorine atom, a chlorine atom, or a bromine atom; a perfluoroalkyl group, such as a trifluoromethyl group; and the like. From the standpoint that the photochromic compound may exhibit a rapid fading rate after being colored by irradiation with light, a fluorine atom or a trifluoromethyl group, which is an electron-withdrawing group, is preferable, and R⁴ is more preferably a fluorine atom or a trifluoromethyl group.

In one embodiment, R³ to R⁶ all may represent hydrogen atoms. In another embodiment, R⁴ may represent a non-conjugated substituent, and R³, R⁵, and R⁶ may be hydrogen atoms; preferably, R⁴ may represent a non-conjugated substituent as mentioned in the above specific examples, and R³, R⁵, and R⁶ may represent hydrogen atoms; and more preferably, R⁴ may represent a fluorine atom or trifluoromethyl group, and R³, R⁵, and R⁶ may represent a hydrogen atom.

R⁷ to R¹⁰ each independently represent a hydrogen atom or a substituent. For substituents, reference may be made to the preceding description.

Both R⁷ are R¹⁰ are preferably electron-donating groups.

In one embodiment, R⁸ and R⁹ may each independently represent a hydrogen atom or an electron-donating group. From the viewpoint of further suppressing the absorption of short-wavelength visible light, it is preferred that R⁸ represents an electron donating group and R⁹ represents hydrogen, or R⁸ and R⁹ independently represent an electron donating group . An electron-donating group represented by R⁸ and an electron-donating group represented by R⁹ may be bonded together to form a ring structure. Examples of such a ring structure may include the following ring structure in which alkyl sulfide groups or alkoxy groups are bonded together, or the following ring structure in which an alkyl sulfide group and an alkoxy group are bonded together. R^{m} described below represents a substituent R^{m} as described previously, preferably a substituted or unsubstituted methyl or ethyl group, and more preferably an unsubstituted methyl or ethyl group.

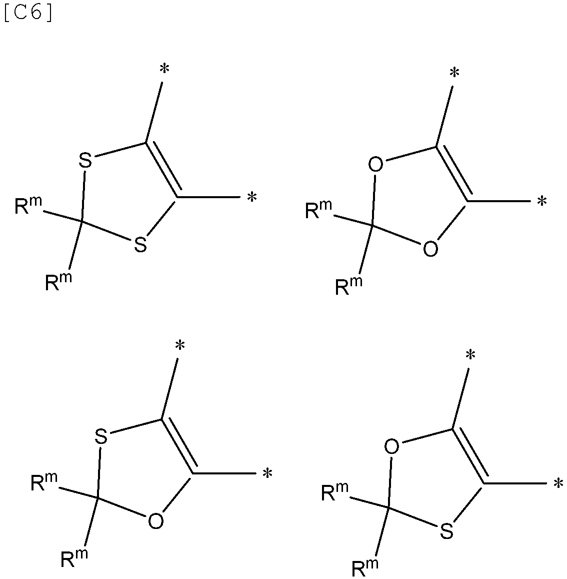

In one embodiment, R⁸ and R⁹ both may represent hydrogen atoms. In another embodiment, one of R⁸ and R⁹ may represent an electron-donating group, and the other may represent a hydrogen atom. In still another embodiment, R⁸ and R⁹ may represent the same or different electron-donating groups. As the electron-donating group represented by R⁸ and/or R⁹, an alkoxy group is preferable, and a methoxy group is more preferable.

In one aspect, R⁸ and R⁹ may each independently represent a hydrogen atom and an electron-withdrawing group. R⁸ and R⁹ each independently representing a hydrogen atom or an electron-withdrawing group are preferable from the viewpoint of further suppressing absorption of short-wavelength visible light. An electron-withdrawing group represented by R⁸ and an electron-withdrawing group represented by R⁹ may be bonded together to form a ring structure. For example, one of R⁸ and R⁹ may represent an electron-withdrawing group and the other may represent a hydrogen atom. In addition, in still another embodiment, R⁸ and R⁹ may represent the same or different electron-withdrawing groups. The electron-withdrawing group represented by R⁸ and/or R⁹ is preferably a halogen atom, and more preferably a fluorine atom. More preferably, R⁹ and R⁴ may represent a fluorine atom; and still more preferably, R⁹ and R⁴ may represent fluorine atoms, and R³ to R⁸ and R¹⁰ may represent hydrogen atoms.

R¹¹ to R²⁰ each independently represent a hydrogen atom or a substituent. It should be noted that at least one of R¹¹ to R²⁰ represents an amino group represented by General Formula 2 below. Having such a structure in which π-conjugate system of the molecular skeleton is extended allows the photochromic compound represented by General Formula 1 to have a large absorption peak on the longer wavelength side. On the other hand, if the conjugate system of the photochromic compound is simply extended, not only the colored form but also the uncolored form tend to absorb light on the longer wavelength side, and absorption of visible light having a short wavelength around 400 nm tends to increase in the state of the colorless form. In contrast, the photochromic compound represented by General Formula 1 having the structure described previously can contribute to the suppression of absorption of visible light having a short wavelength around 400 nm in the state of the colorless form.

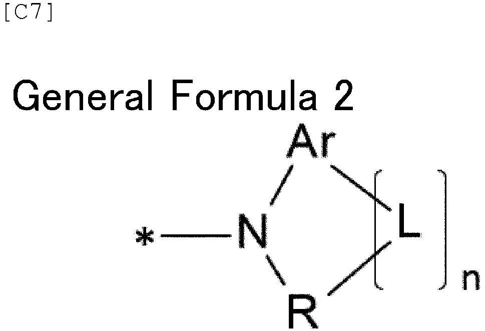

In General Formula 2, * represents a bonding position with a benzene ring.

In General Formula 2, n represents 0 or 1. When n is 0, Ar represents a substituted or unsubstituted aryl group having 5 or more ring constituent atoms, or a substituted or unsubstituted heteroaryl group having 5 or more ring constituent atoms. When n is 1, Ar represents a substituted or unsubstituted arylene group having 5 or more ring constituent atoms, or a substituted or unsubstituted heteroarylene group having 5 or more ring constituent atoms.

The substituted or unsubstituted aryl group having 5 or more ring constituent atoms may be, for example, a substituted or unsubstituted aryl group having 5 or more and 10 or less ring constituent atoms. Specific examples thereof may include a substituted or unsubstituted phenyl group, a substituted or unsubstituted naphthyl group, and the like. When the aryl group is a substituted aryl group, examples of the substituent may include the substituents described previously. Specific examples thereof may include an alkyl group (for example, a methyl group and the like), an alkoxy group (for example, a methoxy group, an ethoxy group, a butoxy group, and the like), an aryl group (for example, a phenoxy group, and the like), a heteroaryl group (for example, a pyridyl group, and the like), and an aryloxy group (for example, a phenoxy group, and the like). The substituents described above may have an additional substituent. Examples of such an additional substituent may include the substituents described previously. A substituent of a substituted aryl group may form a ring structure, such as a condensed ring, together with an aryl group substituted with this substituent. Specific examples of such a ring structure may include ring structures included in exemplary compounds listed below.

The position of substitution of a substituent in the substituted phenyl group may be the ortho, meta, or para position in relation to the nitrogen atom (N) illustrated in General Formula 2, and is preferably the para position.

The substituted or unsubstituted heteroaryl group having 5 or more ring constituent atoms may be, for example, a substituted or unsubstituted heteroaryl group having 5 or more and 10 or less ring constituent atoms. Specific examples thereof may include a substituted or unsubstituted pyridyl group and the like. When the heteroaryl group is a substituted heteroaryl group, reference may be made to the preceding description regarding substituents that the aryl group can have.

With regard to the substituted or unsubstituted arylene group having 5 or more ring constituent atoms, or a substituted or unsubstituted heteroarylene group having 5 or more ring constituent atoms, which are represented by Ar when n is 1, the previous description related to the aryl group and the heteroaryl group for Ar when n is 0 may be applied while replacing the description related to the aryl group with that related to the arylene group, and the description related to the heteroaryl group with that related to the heteroarylene group.

When n is 0, L is not present and R represents a monovalent substituent.

For the monovalent substituent, reference may be made to the preceding description regarding substituents. In one embodiment, the monovalent substituent may represent a substituted or unsubstituted aryl group having 5 or more ring constituent atoms or a substituted or unsubstituted heteroaryl group having 5 or more ring constituent atoms For such aryl and heteroaryl groups, reference may be made to the preceding description regarding Ar. When R represents a substituted or unsubstituted aryl group having 5 or more ring constituent atoms or a substituted or unsubstituted heteroaryl group having 5 or more ring constituent atoms, R may be the same as or different from Ar.

When n is 1, L is present and R represents a divalent substituent. In this case, R is bonded to Ar via L. In one embodiment, the divalent substituent may represent a substituted or unsubstituted arylene group having 5 or more ring constituent atoms or a substituted or unsubstituted heteroarylene group having 5 or more ring constituent atoms. With regard to such arylene and heteroarylene groups, the previous description related to the aryl group and the heteroaryl group for Ar may be applied while replacing the description related to the aryl group with that related to the arylene group, and the description related to the heteroaryl group with that related to the heteroarylene group.

L is a single bond, or a divalent linking group selected from the group consisting of a substituted or unsubstituted, linear, branched, or cyclic alkylene group having 1 to 10 carbon atoms, a substituted or unsubstituted arylene group having 1 to 20 carbon atoms, a substituted or unsubstituted alkylarylene group having 1 to 20 carbon atoms, an ether group (-O-), a sulfide group (-S-), a sulfone group (-S(=O)₂-), a sulfoxide group (-S(=O)-), -NR¹⁰⁰- (where R²⁰⁰ represents a hydrogen atom or a substituent), and -SiRaRb- (where Ra and Rb each independently represent a substituted or unsubstituted alkyl group). When R²⁰⁰ represents a substituent, such a substituent may be, for example, an alkyl group, and preferably a substituted or unsubstituted, linear, branched, or cyclic alkyl group having 1 to 10 (preferably 1 or 2) carbon atoms. Preferably, Ra and Rb may each independently represent a substituted or unsubstituted, linear, branched, or cyclic alkyl group having 1 to 10 (preferably 1 or 2) carbon atoms. When n represents 1 and L is present, the amino group represented by General Formula 2 may be an amino group having a condensed ring structure. Specific examples of such an amino group may include an amino group included in exemplary compounds listed below.

In General Formula 1, at least one of R¹¹ to R²⁰ represents an amino group represented by General Formula 2. When at least two amino groups represented by General Formula 2 are present in R¹¹ to R²⁰, these amino groups may be the same or different amino groups. In the photochromic compound represented by General Formula 1, the total number of amino groups represented by General Formula 2 included as at least one of R¹¹~R²⁰ is 1 or more, and may be 3 or less, or 2 or less. Preferably, among R¹¹ to R²⁰, R¹⁸ may represent an amino group represented by General Formula 2, R¹² and R¹³ may each independently represent a hydrogen atom or a substituent, and the others may represent hydrogen atoms. More preferably, among R¹¹ to R²⁰, R¹⁸ may represent an amino group represented by General Formula 2, R¹³ may represent a substituent, and the others may represent hydrogen atoms. When R¹² and/or R¹³ represent(s) a substituent or substituents, examples of such substituents may include a halogen atom (for example, a fluorine atom), an alkoxy group (for example, a methoxy group, an ethoxy group, a butoxy group, and the like), an alkyl sulfide group (for example, a methylsulfide group and the like), a dialkylamino group (for example, a dimethylamino group and the like), an aryloxy group (for example, a phenoxy group and the like), a cyclic amino group (for example, a piperidino group, a morpholino group, and the like), etc. R1³ may preferably represent an alkoxy group, a dialkylamino group, an aryloxy group, or a cyclic amino group, and more preferably an alkoxy group, a dimethylamino group, a phenoxy group, a piperidino group, or a morpholino group.

In one embodiment, R¹⁸ may represent an amino group represented by General Formula 2, n in General Formula 2 may represent 0, and Ar may represent a substituted aryl group having 5 or more ring constituent atoms. Preferably, R may represent a substituted aryl group having 5 or more ring constituent atoms. More preferably, Ar and R may each independently represent a monosubstituted phenyl group substituted with an alkoxy group. Still more preferably, Ar and R may represent a monosubstituted phenyl group substituted with a methoxy group. The substituted position of the alkoxy or methoxy group is preferably the para position in relation to the nitrogen atom (N) shown in General Formula 2.

Examples of photochromic compounds represented by General Formula 1 may include the following compounds. Specific examples of respective portions in General Formula 1 may also include those included in the following exemplary compounds. However, the present invention is not limited to the following exemplary compounds.

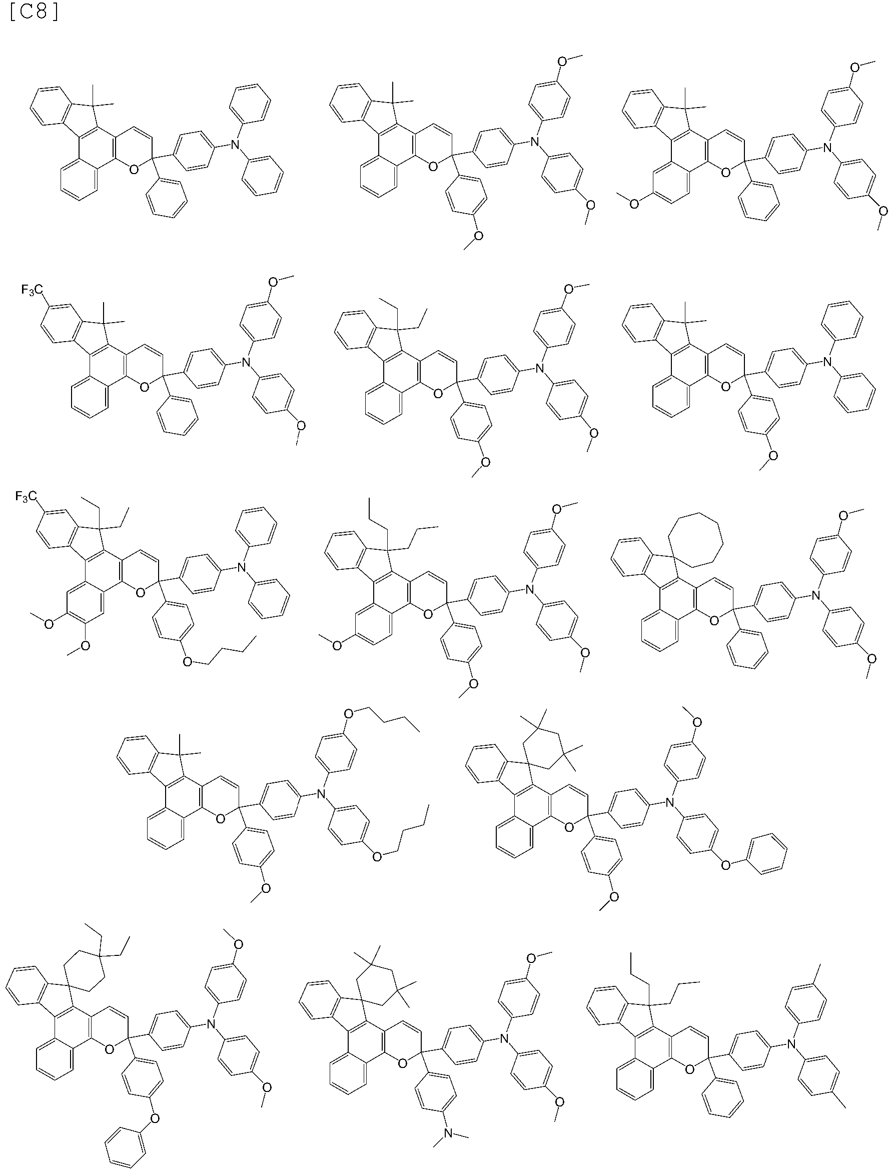

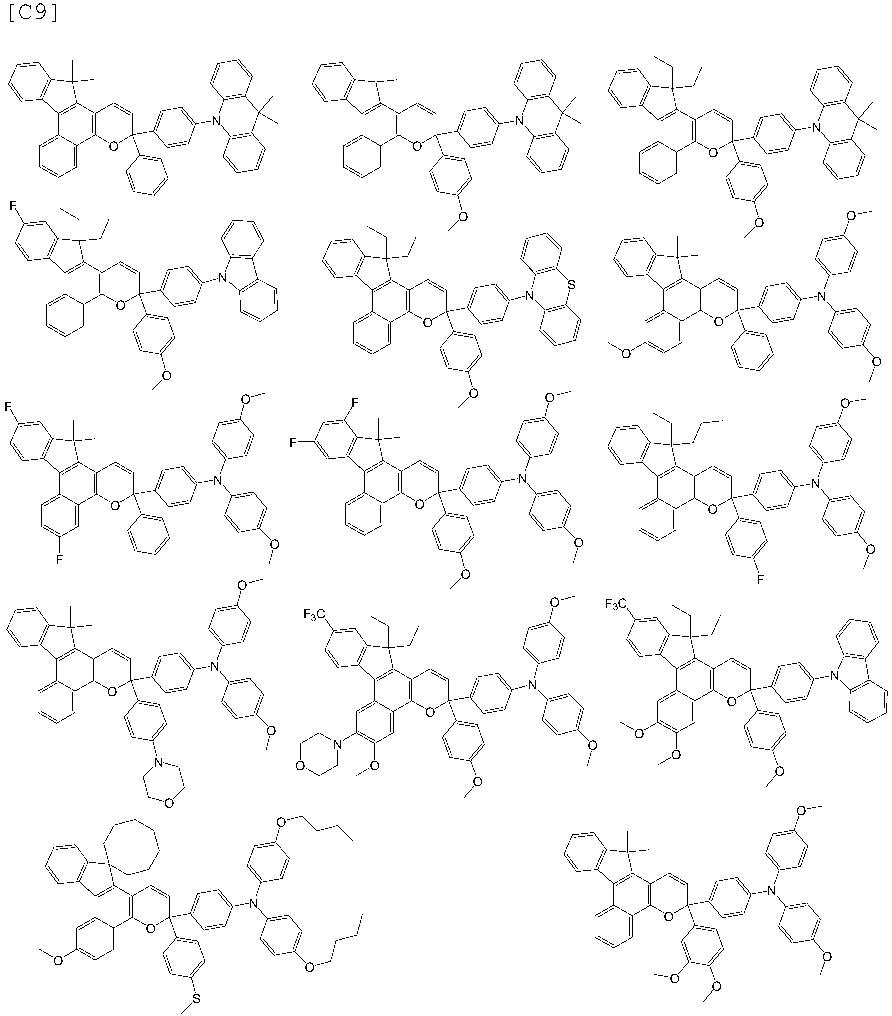

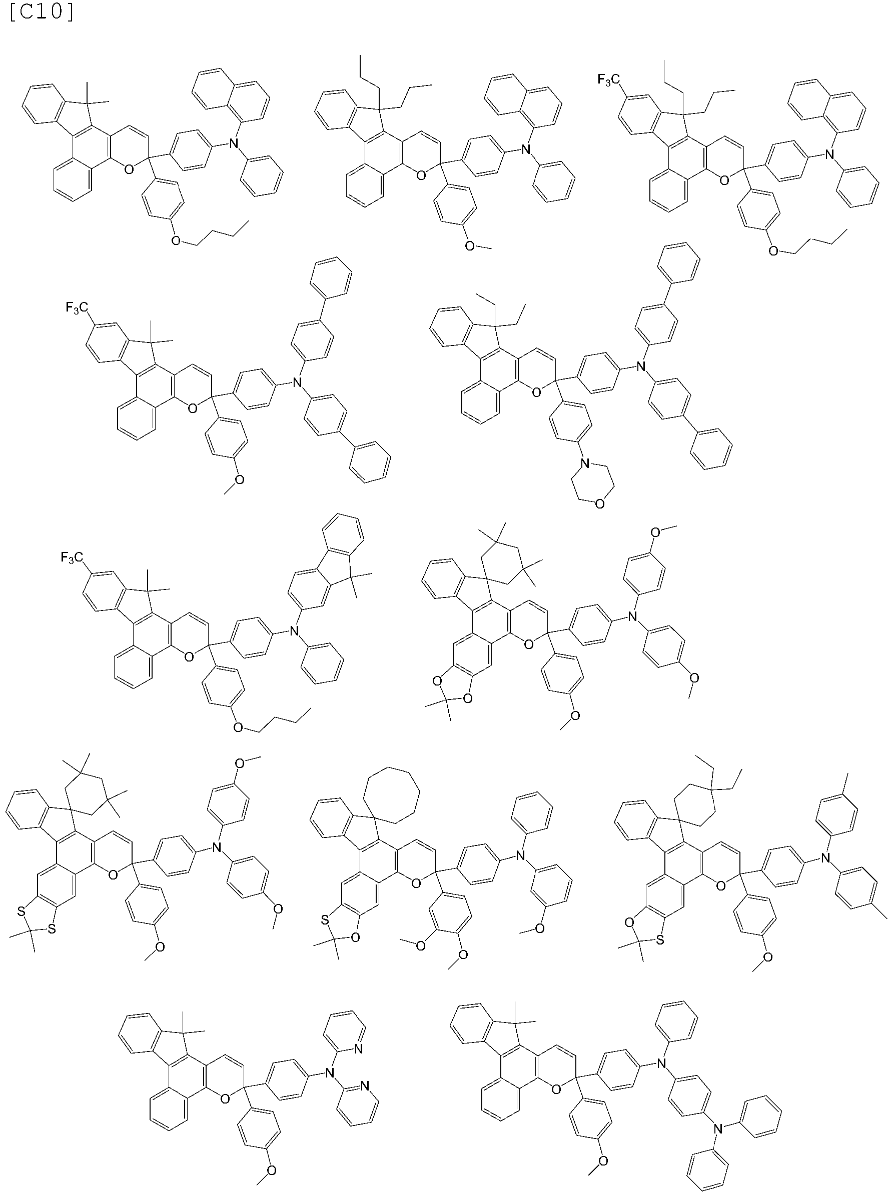

The photochromic compound represented by General Formula 1 may be synthesized by a known method. For the synthesis method, for example, reference may be made to the following documents. Japanese Patent No. 4884578, US 2006/0226402 A1, US 2006/0228557 A1, US 2008/0103301 A1, US 2011/0108781 A1, US 2012/0145973 A1, U.S. Patent Specification No. 7527754, U.S. Patent Specification No. 7556751, WO 2001/60811 A1, WO 2013/086248 A1, WO 1996/014596 A1, and WO 2001/019813 A1.

### [Photochromic Composition and Photochromic Article]

One aspect of the present invention relates to a photochromic composition containing at least one photochromic compound represented by General Formula 1.
In addition, one aspect of the present invention relates to a photochromic article containing at least one photochromic compound represented by General Formula 1.

The photochromic composition and the photochromic article may contain only one photochromic compound represented by General Formula 1, or may contain two or more (for example, two or more and four or less) photochromic compounds represented by General Formula 1. The photochromic article and the photochromic composition may contain, for example, approximately 0.1 to 15.0 mass% of photochromic compounds represented by General Formula 1 relative to the total amount of 100 mass% of the photochromic article or the photochromic composition. However, the present invention is not limited to the above range.

The photochromic article may have at least a substrate. In one embodiment, the photochromic compound represented by General Formula 1 may be contained in the substrate of the photochromic article. The photochromic article may have a substrate and a photochromic layer, and the substrate and/or the photochromic layer may contain at least one photochromic compound represented by General Formula 1. In the substrate and the photochromic layer, the photochromic compound represented by General Formula 1 may be contained only in the substrate in one embodiment, and the photochromic compound may be contained only in the photochromic layer in another embodiment. In still another embodiment, the photochromic compound may be contained in both the substrate and the photochromic layer. In addition, the substrate and the photochromic layer may contain, as a photochromic compound, only the photochromic compound represented by General Formula 1 or may contain at least one other photochromic compound. Examples of other photochromic compounds may include azobenzenes, spiropyrans, spirooxazines, naphthopyrans, indenonaphthopyrans, phenanthropyrans, hexaarylbisimidazoles, donor-acceptor Stenhouse adducts (DASAs), salicylidene anilines, dihydropyrenes, anthracene dimers, fulgides, diarylethenes, phenoxynaphthacenequinones, stilbenes, and the like.

### <Substrate>

The photochromic article may contain a substrate selected according to the type of the photochromic article. Examples of substrates may include spectacle lens substrates, such as a plastic lens substrate and a glass lens substrate. The glass lens substrate may be, for example, a lens substrate made of inorganic glass. Examples of plastic lens substrates may include a (meth)acrylic resin, a styrene resin, a polycarbonate resin, an allyl resin, an allyl carbonate resin, such as a diethylene glycol bisallyl carbonate resin (CR-39), a vinyl resin, a polyester resin, a polyether resin, a urethane resin obtained by the reaction between an isocyanate compound and a hydroxy compound, such as diethylene glycol, a thiourethane resin obtained by the reaction between an isocyanate compound and a polythiol compound, and a cured product (generally referred to as a transparent resin) obtained by curing a curable composition containing a (thio)epoxy compound having at least one disulfide bond in the molecule. As lens substrates, an unstained substrate (colorless lenses) may be used, or a stained substrate (stained lenses) may be used. The refractive index of a lens substrate may be, for example, approximately 1.50 to 1.75. However, the refractive index of the lens substrate is not limited to the range described above, and may be within, above, or below the range described above. Here, the refractive index shall refer to a refractive index for light having a wavelength of 500 nm. In addition, the lens substrate may be a lens with refractive power (so-called prescription lens) or a lens without refractive power (so-called non-prescription lens).

For example, the photochromic composition may be a polymerizable composition. In the present invention and this specification, the term "polymerizable composition" refers to a composition containing at least one polymerizable compound. A polymerizable composition at least containing at least one photochromic compound represented by General Formula 1 and at least one polymerizable compound may be molded by a known molding method to produce a cured product of such a polymerizable composition. Such a cured product may be included as a substrate in the photochromic article and/or may be included as a photochromic layer. The curing may be performed by irradiation with light and/or a heat treatment. The term "polymerizable compound" refers to a compound having a polymerizable group, and as the polymerization reaction of the polymerizable compound proceeds, the polymerizable composition may be cured to form a cured product. The polymerizable composition may further contain at least one additive (for example, a polymerization initiator and the like).

The spectacle lenses may be various lenses, such as a single focus lens, a multifocal lens, and a progressive power lens. The type of the lens is determined by the surface shape of each opposite side of the lens substrate. In addition, the surface of the lens substrate may be any of a convex surface, a concave surface, or a flat surface. In a general lens substrate and spectacle lens, the object-side surface is a convex surface, and the eyeball-side surface is a concave surface. However, the present invention is not limited thereto. Generally, the photochromic layer may be provided on the object-side surface of the lens substrate, but may be provided on the eyeball-side surface.

### <Photochromic Layer>

The photochromic layer may be a layer that is directly provided on the surface of the substrate or indirectly provided via at least one other layer. The photochromic layer may be, for example, a cured layer obtained by curing a polymerizable composition. A photochromic layer may be formed as a cured layer obtained by curing a polymerizable composition at least containing at least one photochromic compound represented by General Formula 1 and at least one polymerizable compound. For example, such a polymerizable composition is directly applied to the surface of the substrate or applied to the surface of a layer provided on the substrate, and the applied polymerizable composition is cured, thereby forming a photochromic layer as a cured layer containing at least one photochromic compound represented by General Formula 1. As the application method, known coating methods, such as a spin coating method, a dip coating method, a spray coating method, an inkjet method, a nozzle coating method, and a slit coating method may be used. The curing may be performed by irradiation with light and/or a heat treatment. The polymerizable composition may further contain at least one additive (for example, a polymerization initiator) in addition to at least one polymerizable compound. As the polymerization reaction of the polymerizable compound proceeds, the polymerizable composition can be cured to form a cured layer.

The thickness of the photochromic layer may be, for example, 5 µm or more, 10 µm or more, or 20 µm or more, and may be, for example, 80 µm or less, 70 µm or less or 50 µm or less.

### <Polymerizable Compound>

In the present invention and this specification, the term "polymerizable compound" shall refer to a compound having at least one polymerizable group in one molecule, and the term "polymerizable group" shall refer to a reactive group that can undergo a polymerization reaction. Examples of polymerizable groups may include an acryloyl group, a methacryloyl group, a vinyl group, a vinyl ether group, an epoxy group, a thiol group, an oxetane group, a hydroxy group, a carboxy group, an amino group, and an isocyanate group.

Examples of polymerizable compounds that can be used to form the above substrate and the above photochromic layer may include the following compounds.

### (Episulfide Compound)

The episulfide compound is a compound having two or more episulfide groups in one molecule. The episulfide group is a polymerizable group that can undergo ring-opening polymerization. Specific examples of episulfide compounds may include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, 2-[1,1-bis(2,3-epithiopropyldithiomethylthio)methyl]-1,3-dithietane, and the like.

### (Thietanyl Compound)

A thietanyl compound is a thietane compound having two or more thietanyl groups in one molecule. A thietanyl group is a polymerizable group that can undergo ring-opening polymerization. Some thietanyl compounds have an episulfide group together with a plurality of thietanyl groups. Such compounds are listed as examples of the episulfide compounds described above. Other thietanyl compounds include metal-containing thietane compounds having metal atoms in the molecule and non-metallic thietane compounds that contain no metal.

Specific examples of non-metallic thietane compounds may include bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanylsulfide, bis(thietanylthio)methane, 3-[<(thietanylthio)methylthio>methylthio]thietane, bisthietanyldisulfide, bisthietanyltrisulfide, bisthietanyltetrasulfide, bisthietanylpentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, 1,1,2,2-tetrakis(3-thietanyldithiomethylthio)ethane, and the like.

Examples of metal-containing thietane compounds may include those containing Group 14 atoms, such as an Sn atom, a Si atom, a Ge atom, and a Pb atom, Group 4 elements, such as a Zr atom and a Ti atom, Group 13 atoms, such as an Al atom, and Group 12 atoms, such as an Zn atom, as metal atoms in the molecule. Specific examples thereof may include an alkylthio(thietanylthio)tin, a bis(alkylthio)bis(thietanylthio)tin, an alkylthio(alkylthio)bis(thietanylthio)tin, a bis(thietanylthio) cyclic dithiotin compound, an alkyl(thietanylthio)tin compound, and the like.

Specific examples of alkylthio(thietanylthio)tins may include methylthiotris(thietanylthio)tin, ethylthiotris(thietanylthio)tin, propylthiotris(thietanylthio)tin, isopropylthiotris(thietanylthio)tin, and the like.

Specific examples of bis(alkylthio)bis(thietanylthio)tins may include bis(methylthio)bis(thietanylthio)tin, bis(ethylthio)bis(thietanylthio)tin, bis(propylthio)bis(thietanylthio)tin, bis(isopropylthio)bis(thietanylthio)tin, and the like.

Specific examples of alkylthio(alkylthio)bis(thietanylthio)tins may include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, isopropylthio(propylthio)bis(thietanylthio)tin, and the like.

Specific examples of bis(thietanylthio) cyclic dithiotin compounds may include bis(thietanylthio)dithiastannetane, bis(thietanylthio)dithiastannolane, bis(thietanylthio)dithiastanninane, bis(thietanylthio)trithiastannocane, and the like.

Specific examples of alkyl(thietanylthio)tin compounds may include methyltris(thietanylthio)tin, dimethyl bis(thietanylthio)tin, butyltris(thietanylthio)tin, tetrakis(thietanylthio)tin, and the like.

### (Polyamine Compound)

The polyamine compound is a compound having two or more NH₂ groups in one molecule, and can form a urea bond through a reaction with a polyisocyanate and can form a thiourea bond through a reaction with a polyisothiocyanate. Specific examples of polyamine compounds may include ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine, 1,3,5-benzenetriamine, and the like.

### (Epoxy-based Compound)

The epoxy-based compound is a compound having an epoxy group in the molecule. The epoxy group is a polymerizable group that can undergo ring-opening polymerization. The epoxy compounds are generally classified into aliphatic epoxy compounds, alicyclic epoxy compounds, and aromatic epoxy compounds.

Specific examples of aliphatic epoxy compounds may include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, tris(2-hydroxyethyl)isocyanurate triglycidyl ether, and the like.

Specific examples of alicyclic epoxy compounds may include isophorone diol diglycidyl ether, bis-2,2-hydroxycyclohexylpropane diglycidyl ether, and the like.

Specific examples of aromatic epoxy compounds may include resorcine diglycidyl ether, bisphenol A diglycidyl ether, bisphenol F diglycidyl ether, bisphenol S diglycidyl ether, orthophthalic acid diglycidyl ester, phenol novolac polyglycidyl ether, cresol novolac polyglycidyl ether, and the like.

In addition to the above examples, an epoxy compound having a sulfur atom in the molecule together with an epoxy group may be used. Such sulfur atom-containing epoxy compounds include linear aliphatic compounds and cycloaliphatic compounds.

Specific examples of linear aliphatic sulfur atom-containing epoxy compounds may include bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, 2,2-bis(2,3-epoxypropylthiomethyl)-1-(2,3-epoxypropylthio)butane, and the like.

Specific examples of cyclic aliphatic sulfur atom-containing epoxy compounds may include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, 2,5-bis(2,3-epoxypropylthiomethyl)-2,5-dimethyl-1,4-dithiane, and the like.

### (Compound Having Radically Polymerizable Group)

The radically polymerizable group is a polymerizable group that can undergo radical polymerization. Examples of radically polymerizable groups may include an acryloyl group, a methacryloyl group, an allyl group, a vinyl group, and the like.

Hereinafter, a compound having a polymerizable group selected from a group consisting of an acryloyl group and a methacryloyl group will be referred to as a "(meth)acrylate compound". Specific examples of (meth)acrylate compounds may include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl(meth)acrylate, bisphenol A di(meth)acrylate, 2,2-bis(4-(meth)acroxyethoxyphenyl)propane, 2,2-bis(4-(meth)acroxydiethoxyphenyl)propane, 2,2-bis(4-(meth)acryloyloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bispheno-F di(meth)acrylate, 1,1-bis(4-(meth)acroxyethoxyphenyl)methane, 1,1-bis(4-(meth)acroxydiethoxyphenyl)methane, dimethyloltricyclodecane di(meth)acrylate; trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methyl thio(meth)acrylate, phenyl thio(meth)acrylate, benzyl thio(meth)acrylate, xylylenedithiol di(meth)acrylate, mercaptoethyl sulfide di(meth)acrylate, bifunctional urethane (meth)acrylate, and the like.

Specific examples of compounds having an allyl group (allyl compounds) may include allyl glycidyl ether, diallyl phthalate, diallyl terephthalate, diallyl isophthalate, diallyl carbonate, diethylene glycol bis(allyl carbonate), methoxypolyethylene glycol allyl ether, polyethylene glycol allyl ether, methoxypolyethyleneglycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, methacryloyloxy polyethylene glycol allyl ether, and the like.

Examples of compounds having a vinyl group (vinyl compounds) may include α-methylstyrene, an α-methylstyrene dimer, styrene, chlorostyrene, methylstyrene, bromostyrene, dibromostyrene, divinylbenzene, 3,9-divinylspirobi(m-dioxane), and the like.

The photochromic article may include, at any position, at least one layer known as a functional layer for the photochromic article, such as a protective layer for improving the durability of the photochromic article, an anti-reflection layer, a water-repellent or hydrophilic anti-fouling layer, an anti-fogging layer, and a primer layer for improving adhesion between layers.

The photochromic article may be an optical article. One form of the optical article is a spectacle lens. Such a spectacle lens may also be called a photochromic lens or a photochromic spectacle lens. In addition, as one form of the optical article, a goggle lens, a visor (bill) part of a sun visor, a shield component of a helmet, and the like may be exemplified. An optical article with anti-glare functionality may be produced by applying the photochromic composition, which is a polymerizable composition, onto a substrate for these optical articles and curing the applied composition to form a photochromic layer.

### [Spectacles]

One aspect of the present invention relates to spectacles having a spectacle lens that is one form of the photochromic article. Details of the spectacle lens included in the spectacles are as described previously. By providing such a spectacle lens, for example, the spectacles can exhibit an anti-glare effect like sunglasses when the photochromic compound is colored upon irradiation with sunlight outdoors, and the photochromic compound can fade upon returning indoors, thereby recovering the transmittance. For the spectacles, a known technology may be applied to the configuration of the frame or the like.

### Examples

Hereinafter, the present invention will be described in more detail referring to Examples. However, the present invention is not limited to the embodiments shown in Examples.

### [Example 1]

From the raw material compound A and the raw material compound B shown in Table 1, a product shown in Table 1 was obtained by the following method.
Under an argon atmosphere, the raw material compound A (125 g, 243 mmol), dipalladium-tris(dibenzylideneacetone)chloroform complex (Pd₂dba₃·CHCl₃, 2.52 g, 2.43 mmol), XPhos (2-dicyclohexylphosphino-2',4',6'-triisopropylbiphenyl) (2.52 g, 2.43 mmol), tert-butoxysodium (32.7 g, 341 mmol), and a solution of diphenylamine (61.8 g, 365 mmol) in toluene (300 mL) as the raw material compound B was stirred at external temperature of 105°C for 2 hours. After cooling, the resulting mixture was filtered through celite, and the insoluble matter was washed with chloroform (500 mL). The concentrate of the oven solution was purified by silica gel column chromatography (SiO₂: 2 kg, heptane/ethyl acetate (on volume basis) = 90/10 to 75/25) to yield a product (120 g) as a dark blue solid.

The molecular structure of the product was identified by nuclear magnetic resonance apparatus (proton NMR), precision mass spectrometry, and CHN elemental analysis.
As the nuclear magnetic resonance apparatus, ECS-400, manufactured by JEOL Ltd., was used. As the measurement solvent, deuterochloroform or deuterated dimethyl sulfoxide was used.
For the precision mass spectrometry, a system including a high-performance liquid chromatography LC-30A system, manufactured by Shimadzu Corporation, equipped with a Q Exactive mass spectrometer, manufactured by Thermo Fisher Scientific Corporation, was used.
A combustion method was used for CHN elemental analysis. Purities were analyzed using high-performance liquid chromatography (HPLC). As HPLC, LC-2040C (manufactured by Shimadzu Corporation) was used for purity analysis.
It was comprehensively confirmed based on the above analysis results that the product shown in Table 1, which was a target compound, was obtained.

### [Examples 2 to 6 and Comparative Examples 1 and 2]

Products shown in Table 1 were obtained by the same operation, except that the compounds shown in Table 1 were used as the raw material compound A and the raw material compound B that were used to synthesize compounds.
The obtained products were analyzed by the method described previously. It was comprehensively confirmed based on the above analysis results that the products shown in Table 1, which were target compounds, were obtained.

### [Evaluation Method]

### <Evaluation of Transparency of Uncolored Form in Short-Wavelength Visible Region>

Each compound of Examples and Comparative Examples was dissolved in chloroform containing no stabilizer to prepare a chloroform solution containing the compound (concentration: 2.8 × 10⁻³ mol/L).
A 1 cm square quartz spectroscopic cell containing the prepared solution was covered, then left in a UV-visible spectrophotometer (that is, under light-shielded conditions) for 30 minutes, and the absorbance was then measured using a UV-visible spectrophotometer (UV-1900i, manufactured by Shimadzu Corporation, measurement wavelength: 800 to 250 nm, wavelength increments: 2 nm, high-speed mode). The absorbance was measured at room temperature (20°C to 25°C).
The molar absorption coefficient ε (mol⁻¹Lcm⁻¹) at a wavelength of 400 nm was read. When the molar absorption coefficient ε was less than 6,000 mol⁻¹Lcm⁻¹, it was judged as "satisfactory", and when the molar absorption coefficient ε was 6,000 mol⁻¹Lcm⁻¹ or more, it was judged as "unsatisfactory".
The compounds of Examples 1 to 6 and the compound of Comparative Example 2 all had molar extinction coefficients ε at a wavelength of 400 nm of 6000 mol⁻¹Lcm⁻¹, which were judged as "satisfactory". The compound of Comparative Example 2 had a molar absorption coefficient ε at a wavelength of 400 nm of 2900 mol⁻¹Lcm⁻¹.
The compound of Comparative Example 1 had a molar extinction coefficient ε at a wavelength of 400 nm of 6200 mol⁻¹Lcm⁻¹, which was judged as " unsatisfactory ".

### <Evaluation of Absorption Characteristics of Colored Form>

For each of Examples and Comparative Examples shown in Table 1, each compound was dissolved in chloroform containing no stabilizer to prepare a chloroform solution containing the compound.
A 1 cm square quartz spectroscopic cell containing the prepared solution was covered, and irradiated with ultraviolet rays for 15 seconds using UV-LED (a combination of LIGHTNINGCURE LC-L1V5 and L14310-120, output: 70%), manufactured by Hamamatsu Photonics K.K., as the ultraviolet light source. The solution was stirred with a small stirrer during the irradiation with ultraviolet rays. Within 10 seconds after the irradiation with ultraviolet rays was completed, the absorbance was measured using a UV-visible spectrophotometer (UV-1900i manufactured by Shimadzu Corporation, measurement wavelength: 700 to 400 nm, wavelength increments: 2 nm, survey mode) to obtain an absorption spectrum. The absorbance was measured at room temperature (23°C to 28°C). The solution was prepared to have a concentration such that the absorbance at the first absorption wavelength (the peak of the absorption intensity observed at the longest wavelength) was 0.95 to 1.05.
The wavelength of the first absorption wavelength (the peak of the absorption intensity observed at the longest wavelength) in the absorption spectrum thus obtained was read. The read wavelengths are shown as "maximum absorption peak wavelength" in Table 1. From the viewpoint of the usefulness as a photochromic compound having a large absorption peak on the longer wavelength side, the maximum absorption peak wavelength thus obtained is preferably a wavelength of 580 nm or greater, and more preferably 600 nm or greater.

The above results are shown in Table 1 (Tables 1-1 to 1-3).

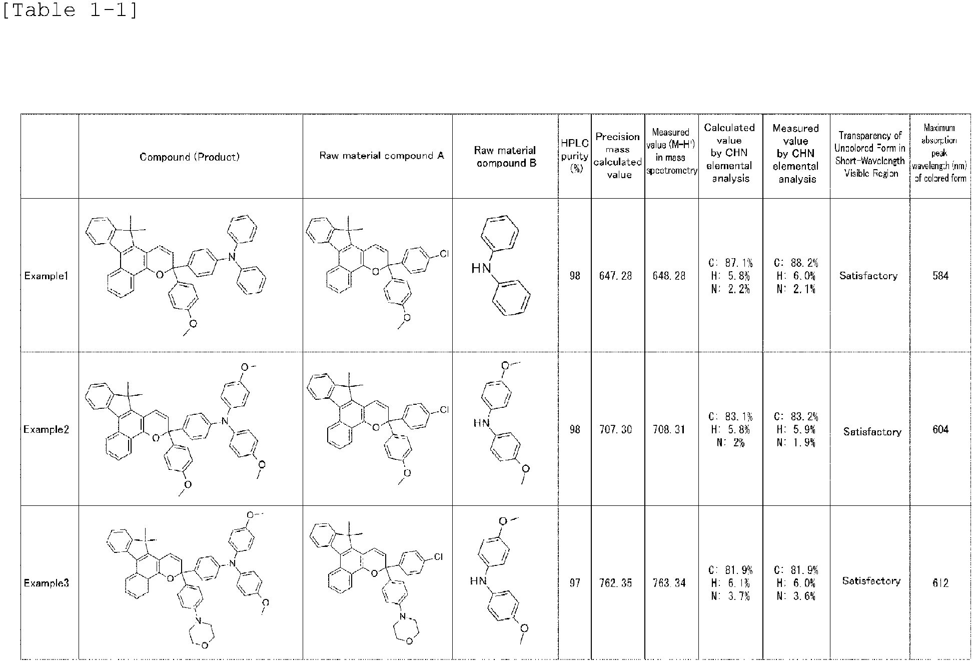

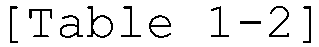

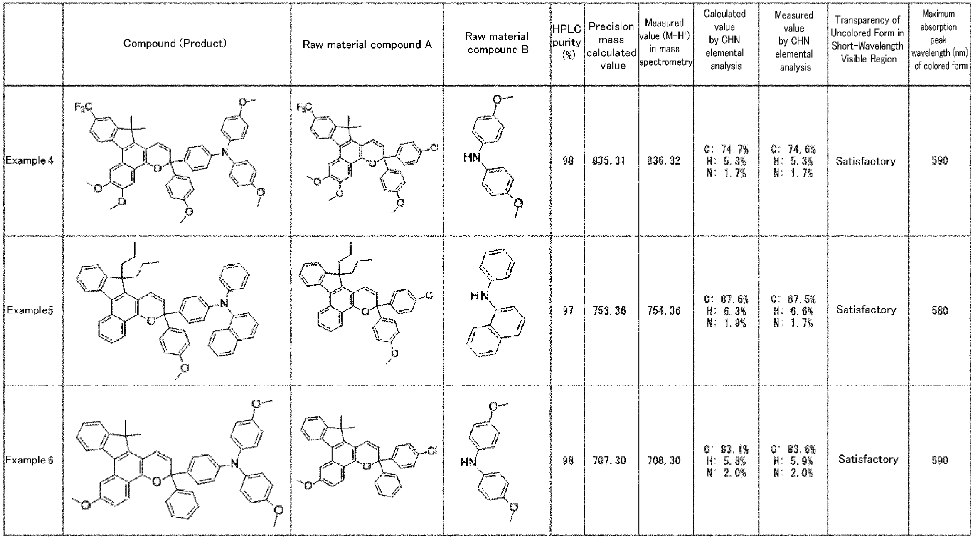

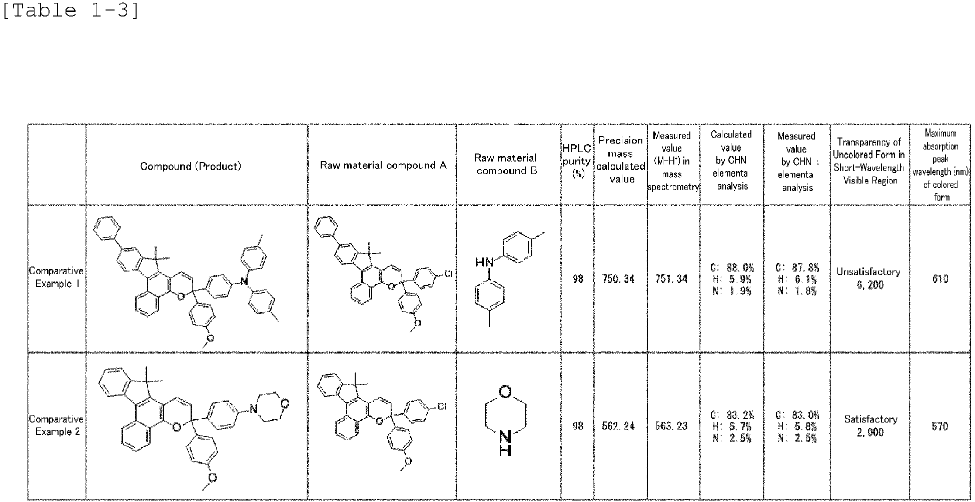

Finally, the above embodiments are summarized.
[1] A photochromic compound represented by General Formula 1 below: 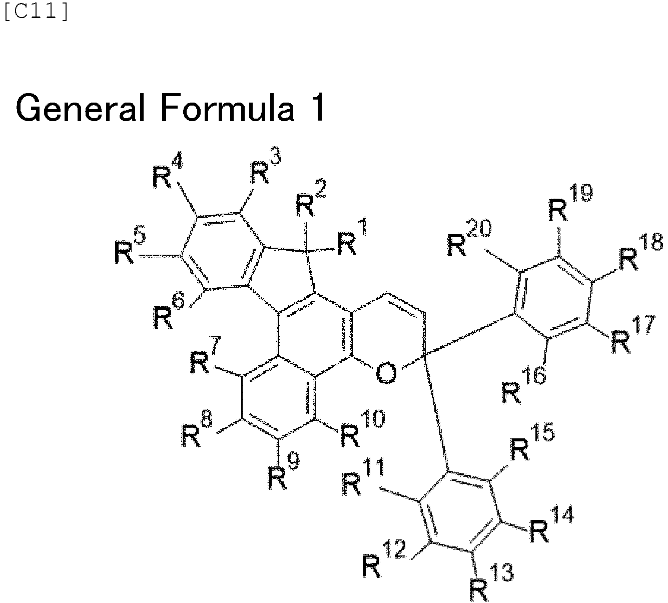
   (in General Formula 1,
   R¹ and R² are bonded together to form a ring structure, the ring structure being a ring structure spiro-condensed with an indeno-condensed naphthopyran, or
   R¹ and R² each independently represent a substituted or unsubstituted, linear or branched alkyl group having 1 or more carbon atoms; a substituted or unsubstituted cyclic alkyl group having 3 or more carbon atoms; or a substituted or unsubstituted group represented by -(R¹⁰⁰)ₙR¹⁰¹, where R¹⁰⁰ represents an alkyleneoxy group, R¹⁰¹ represents an alkyl group, n represents an integer of 1 or more, and a total number of carbon atoms in n alkyleneoxy groups represented by R¹⁰⁰ and an alkyl group represented by R¹⁰¹ is 2 or more;
   R³ and R⁶ each independently represent a hydrogen atom or a non-conjugated substituent;
   R⁷ to R¹⁰ each independently represent a hydrogen atom or a substituent;
   R¹¹ to R²⁰ each independently represent a hydrogen atom or a substituent, provided that at least one of R¹¹ to R²⁰ represents an amino group represented by General Formula 2 below); 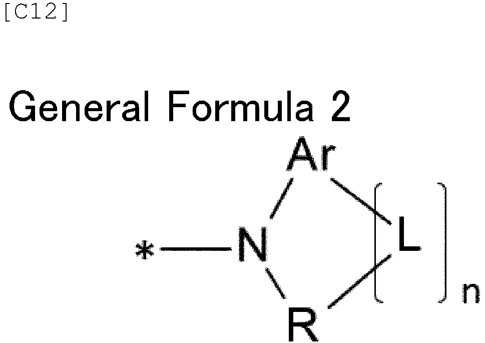
   (in General Formula 2,
   n represents 0 or 1;
   when n is 0, Ar represents a substituted or unsubstituted aryl group having 5 or more ring constituent atoms, or a substituted or unsubstituted heteroaryl group having 5 or more ring constituent atoms, and when n is 1, Ar represents a substituted or unsubstituted arylene group having 5 or more ring constituent atoms, or a substituted or unsubstituted heteroarylene group having 5 or more ring constituent atoms;
   when n is 0, R represents a monovalent substituent, and when n is 1, R represents a divalent substituent;
   L is a single bond or a divalent linking group selected from the group consisting of a substituted or unsubstituted, linear, branched, or cyclic alkylene group having 1 to 10 carbon atoms, a substituted or unsubstituted arylene group having 1 to 20 carbon atoms, a substituted or unsubstituted alkylarylene group having 1 to 20 carbon atoms, an ether group, a sulfide group, a sulfone group, a sulfoxide group, - NR²⁰⁰- (where R²⁰⁰ represents a hydrogen atom or a substituent), and -SiRaRb - (where Ra and Rb each independently represent a substituted or unsubstituted alkyl group); and
   * represents a bonding position with a benzene ring).
[2] The photochromic compound according to [1], wherein, in General Formula 2, n represents 0, and R represents a substituted or unsubstituted aryl group having 5 or more ring constituent atoms or a substituted or unsubstituted heteroaryl group having 5 or more ring constituent atoms.
[3] The photochromic compound according to [1], wherein, in General Formula 2, n represents 1, and R represents a substituted or unsubstituted arylene group having 5 or more ring constituent atoms or a substituted or unsubstituted heteroarylene group having 5 or more ring constituent atoms.
[4] The photochromic compound according to any one of [1] to [3], wherein among R¹¹ to R²⁰ in General Formula 1,
   R¹⁸ represents an amino group represented by General Formula 2;
   R¹² and R¹³ each independently represent a hydrogen atom or a substituent; and
   the others represent hydrogen atoms
[5] The photochromic compound according to any one of [1] to [3], wherein among R¹¹ to R²⁰ in General Formula 1,
   R¹⁸ represents an amino group represented by General Formula 2;
   R¹³ represents a substituent; and
   the others represent hydrogen atoms.
[6] The photochromic compound according to [5], wherein R¹³ represents an alkoxy group, a dimethylamino group, a phenoxy group, a piperidino group, or a morpholino group.
[7] The photochromic compound according to [6], wherein R¹⁸ represents an amino group represented by General Formula 2, and
   in General Formula 2, n represents 0, and Ar represents a substituted aryl group having 5 or more ring constituent atoms.
[8] The photochromic compound according to [7], wherein, in General Formula 2, R represents a substituted aryl group having 5 or more ring constituent atoms.
[9] The photochromic compound according to any one of [1] to [8], wherein, in General Formula 1, R⁸ and R⁹ each independently represent a hydrogen atom or an electron-donating group
[10] The photochromic compound according to any one of [1] to [8], wherein, in General Formula 1, R⁴ and R⁹ both represent a fluorine atom.
[11] A photochromic composition including the photochromic compound as defined in any one of [1] to [10].
[12] The photochromic compound according to [11], further including a polymerizable compound.
[13] A photochromic article including a cured product obtained by curing the photochromic composition as defined in [12] .
[14] The photochromic article according to [13], including a substrate, and a photochromic layer which is the cured product.
[15] The photochromic article according to [13] or [14, which is spectacle lens.
[16] The photochromic article according to [13] or [14], which is a goggle lens, a visor part of a sun visor, or a shield member of a helmet.
[17] Spectacles including the spectacle lens as defined in [15].

Two or more of the various embodiments and various forms described in this specification may be combined in any combination.

The embodiment disclosed should be considered illustrative in all respects and not restrictive. The scope of the present invention is shown not by the above description but by the scope of the claims, and is intended to encompass all modifications within equivalent meaning and scope to the scope of the claims.

### Industrial Applicability

One aspect of the present invention is beneficial in the technical fields of spectacles, goggles, sun visors, helmets, and the like.

## Claims

1. A photochromic compound represented by General Formula 1 below: 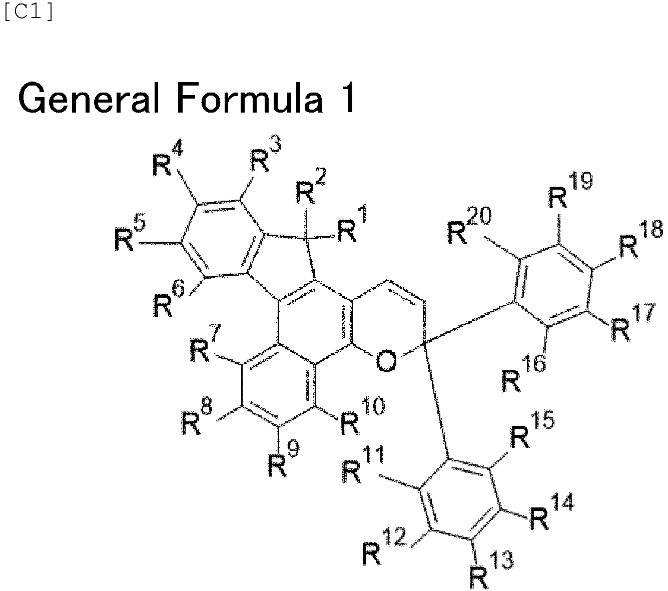
(in General Formula 1,
R¹ and R² are bonded together to form a ring structure, the ring structure being a ring structure spiro-condensed with an indeno-condensed naphthopyran, or
R¹ and R² each independently represent a substituted or unsubstituted, linear or branched alkyl group having 1 or more carbon atoms; a substituted or unsubstituted cyclic alkyl group having 3 or more carbon atoms; or a substituted or unsubstituted group represented by - (R¹⁰⁰)ₙR¹⁰¹, where R¹⁰⁰ represents an alkyleneoxy group, R¹⁰¹ represents an alkyl group, n represents an integer of 1 or more, and a total number of carbon atoms in n alkyleneoxy groups represented by R¹⁰⁰ and an alkyl group represented by R¹⁰¹ is 2 or more;
R³ to R⁶ each independently represent a hydrogen atom or a non-conjugated substituent;
R⁷ to R¹⁰ each independently represent a hydrogen atom or a substituent;
R¹¹ to R²⁰ each independently represent a hydrogen atom or a substituent, provided that at least one of R¹¹ to R²⁰ represents an amino group represented by General Formula 2 below); 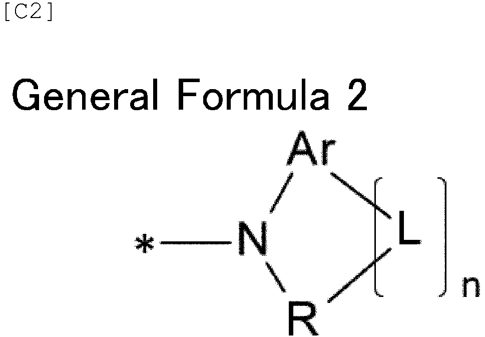
[in General Formula 2,
n represents 0 or 1;
when n is 0, Ar represents a substituted or unsubstituted aryl group having 5 or more ring constituent atoms, or a substituted or unsubstituted heteroaryl group having 5 or more ring constituent atoms, and when n is 1, Ar represents a substituted or unsubstituted arylene group having 5 or more ring constituent atoms, or a substituted or unsubstituted heteroarylene group having 5 or more ring constituent atoms;
when n is 0, R represents a monovalent substituent, and when n is 1, R represents a divalent substituent;
L is a single bond or a divalent linking group selected from the group consisting of a substituted or unsubstituted, linear, branched, or cyclic alkylene group having 1 to 10 carbon atoms, a substituted or unsubstituted arylene group having 1 to 20 carbon atoms, a substituted or unsubstituted alkylarylene group having 1 to 20 carbon atoms, an ether group, a sulfide group, a sulfone group, a sulfoxide group, - NR²⁰⁰- (where R²⁰⁰ represents a hydrogen atom or a substituent), and -SiRaRb - (where Ra and Rb each independently represent a substituted or unsubstituted alkyl group); and
* represents a bonding position with a benzene ring].

2. The photochromic compound according to claim 1, wherein, in General Formula 2, n represents 0, and R represents a substituted or unsubstituted aryl group having 5 or more ring constituent atoms or a substituted or unsubstituted heteroaryl group having 5 or more ring constituent atoms.

3. The photochromic compound according to claim 1, wherein, in General Formula 2, n represents 1, and R represents a substituted or unsubstituted arylene group having 5 or more ring constituent atoms or a substituted or unsubstituted heteroarylene group having 5 or more ring constituent atoms.

4. The photochromic compound according to any one of claims 1 to 3, wherein among R¹¹ to R²⁰ in General Formula 1,
R¹⁸ represents an amino group represented by General Formula 2;
R¹² and R¹³ each independently represent a hydrogen atom or a substituent; and
the others represent hydrogen atoms.

5. The photochromic compound according to any one of claims 1 to 3, wherein among R¹¹ to R²⁰ in General Formula 1,
R¹⁸ represents an amino group represented by General Formula 2;
R¹³ represents a substituent; and
the others represent hydrogen atoms.

6. The photochromic compound according to claim 5, wherein R¹³ represents an alkoxy group, a dimethylamino group, a phenoxy group, a piperidino group, or a morpholino group.

7. The photochromic compound according to claim 6, wherein R¹⁸ represents an amino group represented by General Formula 2, and
in General Formula 2, n represents 0, and Ar represents a substituted aryl group having 5 or more ring constituent atoms.

8. The photochromic compound according to claim 7, wherein, in General Formula 2, R represents a substituted aryl group having 5 or more ring constituent atoms.

9. The photochromic compound according to any one of claims 1 to 8, wherein, in General Formula 1, R⁸ and R⁹ each independently represent a hydrogen atom or an electron-donating group.

10. The photochromic compound according to any one of claims 1 to 8, wherein, in General Formula 1, R⁴ and R⁹ both represent a fluorine atom.

11. A photochromic composition comprising the photochromic compound as defined in any one of claims 1 to 10.

12. The photochromic composition according to claim 11, further comprising a polymerizable compound.

13. A photochromic article comprising a cured product obtained by curing the photochromic composition as defined in claim 12.

14. The photochromic article according to claim 13, comprising a substrate, and a photochromic layer which is the cured product.

15. The photochromic article according to claim 13 or 14, which is a spectacle lens.

16. The photochromic article according to claim 13 or 14, which is a goggle lens, a visor part of a sun visor, or a shield component of a helmet.

17. Spectacles comprising the spectacle lens as defined in claim 15.
